# EUROPEAN PATENT SPECIFICATION

(11) **EP 4 115 856 B1**
(45) Date of publication and mention of the grant of the patent: **12.11.2025**
(21) Application number: 22182734.8
(22) Date of filing: 04.07.2022
(51) Int. Cl.: A61F 9/04

(54) **SLEEP MASK AND MANUFACTURING METHOD**
SCHLAFMASKE UND HERSTELLUNGSVERFAHREN
MASQUE DE SOMMEIL ET PROCÉDÉ DE FABRICATION

(30) Priority: 05.07.2021 AU 2021902041
(43) Date of publication of application: 11.01.2023
(73) Proprietor: Slip IP Holdings Pty Ltd, Fortitude Valley, Queensland 4006 (AU)
(72) Inventor: DUBOIS, Justin, Fortitude Valley, 4006 (AU); STEWART, Fiona Margaret, Fortitude Valley, 4006 (AU)
(74) Representative: Dompatent Partnerschaft von Patent- und Rechtsanwälten mbB

(56) References cited:
- WO-A1-2020/041022
- AU-A4- 2016 100 716
- US-A1- 2009 255 026
- US-A1- 2014 331 383
- US-A1- 2016 008 175
- US-A1- 2016 262 936
- US-A1- 2020 306 497

## Description

### FIELD

This disclosure relates to the field of masks, and, in particular, sleep masks. This disclosure also relates to a method of producing a mask.

### CROSS-REFERENCE TO RELATED APPLICATION

This application claims priority to Australian provisional patent application number 2021902041, filed July 5, 2021.

### BACKGROUND

Research has shown that sleep masks can have a positive effect on sleep, promoting melatonin and cortisol levels, and increasing REM (rapid eye movement) sleep. Sleep masks are known to aid in blocking intrusive or undesired light and can be used by people who have (for example): i) trouble falling asleep; ii) insomnia or other sleep disorders; iii) a need to sleep during the day due to (for instance) shift work; or iv) a wish to sleep while travelling, such as on airplanes.

Key features of a sleep mask are the ability to block as much light as possible and the ability to remain in place while the wearer is asleep. However, the technical field of sleep masks is still largely undeveloped and compromises between features such as comfort, shape, the weight of the mask and the material used are unrefined. Dermatological concerns are also commonly overlooked in the design of sleep masks and finer materials are ignored due to complexities in production.

Accommodating for different facial features also remains an undeveloped area for sleep masks. Whilst some sleep masks have attempted to accommodate, for example, eye lash extensions, these look and feel akin to wearing swimming goggles and lack refinement. Furthermore, as these masks fail to appreciate the contours of a face, the eye lash extensions may still contact the sleep mask leading to damage of the eye lash extensions.
In US 2016/008175 A1, a sleep mask system is described comprising a sleep mask with a front and a back. The back of the sleep mask comprises two eye cavities corresponding to the spacing of the user's eyes. The sleep mask system further comprises a gel pad which is removably attachable to the back of the sleep mask. The gel pad comprises eye holes being aligned with the eye cavities of the sleep mask when attached thereto.
In US 2014/331383 A1, a sleeping mask is described which is comprised of a fabric structure with a first concave shape and a second concave shape that fit over the region of each eye of a user. The concave shapes protect the eyes while the user is sleeping.
In US 2009/255026 A1, an eye shade is described comprising a first side, a second side, a first eye portion and a second eye portion. The eye shade further comprises a nose portion formed from the first side and the second side. The described eye shade is configured to prevent light from passing between a user's face and the eye shade.
In US 2020/306497 A1, a sleep mask with aromatherapy capabilities is described. The sleep mask comprises a front side and a back side. The front side and the back side are composed of a layered fabric. The sleep mask further comprises an adjustable strap and eye pockets. Furthermore, the sleep mask comprises a nasal arch having a top side, a first side, and a second side. Moreover, the sleep mask comprises a light shield being attached around the nasal arch on the back side of the sleep mask. The sleep mask also comprises two diffuser holders comprising an interior cavity and an opening, wherein the interior cavity is configured to receive diffuser material.
In AU 2016 100 716 A4, a sleep mask is described comprising an outwardly projecting part of the size and shape and in a position such that it provides a space suitable for accommodating the fully extended eyelashes of the wearer of the sleep mask when the eye is either open or closed.
In WO 2020/041022 A1, a therapeutic eye compress system is described. The therapeutic device is configured to provide moist heat therapy to a body part such as an eye of a human or animal subject. The therapeutic mask comprises a mask body configured to be attached to a user and a treatment pod comprising a loose, granular fill material contained within an outer shell. At least one treatment pod is releasably coupled to the mask body.
In US 2016/262936 A1, a sleep mask apparatus is described for use over a user's eyes. The described sleep mask apparatus includes an opaque element having an inner surface and an opposing outer surface. The opaque element is sized and configured to extend across both of the user's eyes to mitigate the passage of light to broad the user's eyes with the inner surface facing the user and the outer surface facing away from the user.

Bearing this in mind, the present inventor(s) have developed a new mask, which is particularly suitable as a sleep mask.

Any reference to or discussion of any document, act or item of knowledge in this specification is included solely for the purpose of providing a context for the present invention. It is not suggested or represented that any of these matters or any combination thereof formed at the priority date part of the common general knowledge, or was known to be relevant to an attempt to solve any problem with which this specification is concerned.

### SUMMARY

According to the present invention, a mask and a method for producing a mask as defined in the enclosed independent claims are suggested. Further preferred embodiments of the present invention are defined in the dependent claims.

In an embodiment, the two contoured recesses are configured to avoid eyelash extensions. In an embodiment, the two contoured recesses assist in avoiding contact with eyes and/or long eyelashes.

In an embodiment, the one or more padding parts assist in forming the shape of the contoured recesses.

In an embodiment, the outer portion and/or the face portion form a layer. In an embodiment, the layer is at least partly made of silk or satin.

In an embodiment, the retaining part includes silk or satin.

In an embodiment, the contoured recesses include a valley portion.

In an embodiment, the valley portion is substantially flat.

According to the present invention, a recess seam extends through the face portion into the one or more padding portions to define the valley portion.

According to the present invention, the recess seam includes stitching.

In an embodiment, the valley portion is biased to one side of a longitudinal centre line extending across the body.

In an embodiment, the valley portion is asymmetrical.

In an embodiment, the contoured recesses includes an upper ridge and a lower ridge.

In an embodiment, the lower ridge extends further away from an outer portion of the body, in a direction towards the eyes of the wearer, compared to the upper ridge.

In an embodiment, the density of the one or more padding parts associated with at least part of the lower ridge or the upper ridge is lower compared to the density of the one or more padding parts associated with the valley portion.

In an embodiment, the face portion is at least partly offset from the one or more padding parts in a taper portion between lower ridge and the valley portion.

In an embodiment, a seam portion connects the face portion and the outer portion together with the one or more padding parts therebetween.

In an embodiment, the body includes a filler part.

In an embodiment, the filler part takes the form of a layer.

In an embodiment, the filler part includes a filler and/or a liner.

In an embodiment, the filler part is configured to assists with providing comfort to the wearer and/or improving the appearance of the mask.

In an embodiment, the filler part is located between the one or more padding parts and at least one of the face portion or outer portion.

In an embodiment, the seam portion connects the outer portion to the face portion to retain the filler part and the one or more padding parts.

In an embodiment, the seam portion includes stitching.

In an embodiment, the filler part assists in bridging contours between the one or more padding parts and at least one of the outer portion or the face portion.

In an embodiment, the filler part assists in providing support to the outer portion to allow the outer portion to form a shape that is different to the one or more padding parts.

In an embodiment, the filler and/or the liner include silk or satin.

In an embodiment, the silk includes tussah silk or mulberry silk.

In an embodiment, the one or more padding parts are made from foam.

In an embodiment, the one or more padding parts include polyester and/or polyurethane.

In an embodiment, the one or more padding parts include a density of approximately 28kg/m<3> to 38kg/m<3>.

In an embodiment, the surface hardness of the one or more padding parts measures 32 to 55 on an asker durometer type F.

In a further form, a method of producing a mask according to claim 12 is disclosed, the method including the steps of:
moulding two contoured recesses in one or more padding parts to form part of a body, the contoured recesses to be associated with eyes of a wearer when the mask is worn;
locating the one or more padding parts between an outer portion and a face portion;
connecting the outer portion to the face portion; and
connecting a retaining part to the body.

In an embodiment, at least part of the body includes silk or satin.

In an embodiment, the step of connecting the retaining part to the body includes stitching the retaining part to the body.

In an embodiment, the step of shaping the two contoured recesses to form part of the body includes stitching a face portion of the body to one or more padding parts to form a valley portion.

In an embodiment, the method further includes locating a filler part between the one or more padding parts and at least one of the face portion or the outer portion.

In an embodiment, the step of locating the filler part includes locating it between the one or more padding parts and the outer portion of the body.

In an embodiment, the step of connecting the face portion to the outer portion includes stitching the two together.

Further features and advantages of the present disclosure will become apparent from the following detailed description.

### BRIEF DESCRIPTION OF THE DRAWINGS

By way of example only, embodiments of the present disclosure will be described more fully hereinafter with reference to the accompanying figures, wherein:
Figure 1 illustrates a front view of a sleep mask, according to one embodiment;
Figure 2 illustrates a rear view of the sleep mask shown in Figure 1;
Figure 3 illustrates a further rear view of the sleep mask shown in Figure 1 ;
Figure 4 illustrates a perspective view of the sleep mask shown in Figure 1 ;
Figure 5 illustrates an exploded view of the sleep mask shown in Figure 1; and
Figure 6 illustrates a cross sectional view of the sleep mask shown in Figure 4 , along the line 6-6.

### DETAILED DESCRIPTION

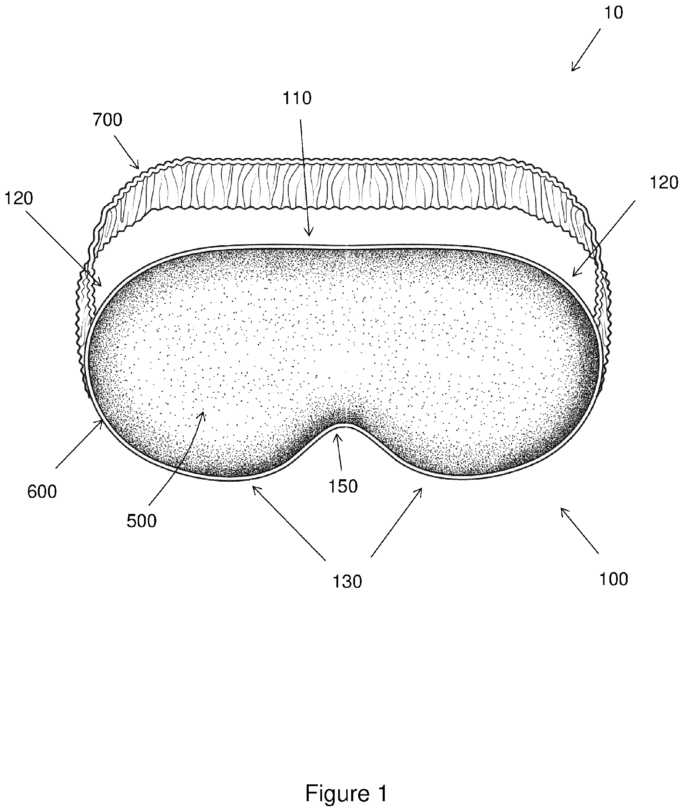

Figure 1 illustrates a front view of a mask in the form of a sleep mask (or eye mask) 10, according to an embodiment of the present disclosure. The sleep mask 10 comprises a body 100 and a retaining part 700 connected to the body 100. The body 100 is adapted to cover the eyes of a wearer of the sleep mask 10 and, as detailed further below, the retaining part 700 is configured to fit over the head of a wearer to retain the body 100 to the wearer.

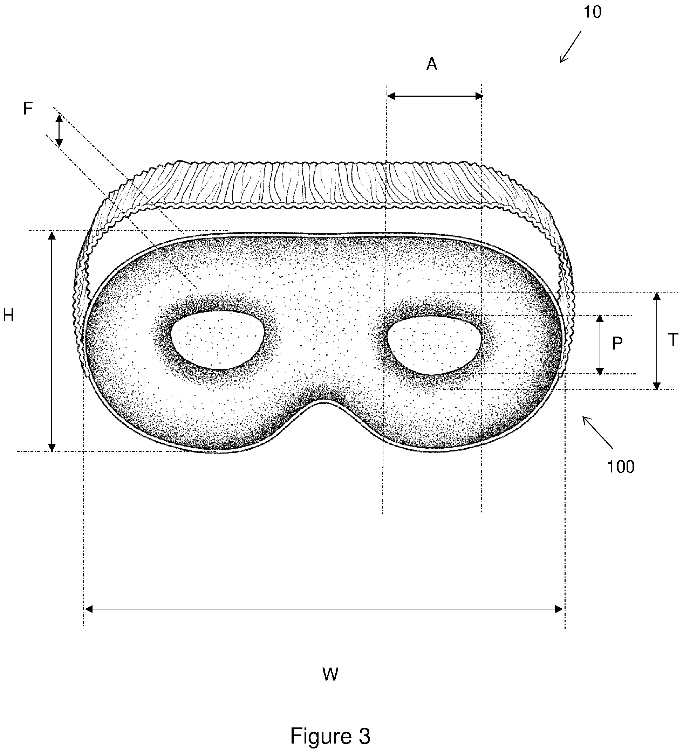

The body 100 has a largely straight edge 110 along the top of the body 100 and smooth curves at opposing sides 120 of the body 100. The bottom of the body 100 has two symmetrical curves 130 that substantially form a w-shape. The two curves 130 have a nose portion 150 spaced between them. The nose portion 150 is designed to sit across the bridge of the wearer's nose. The body 100 has a height (H) of approximately 96 mm and a width (W) of approximately 210 mm (as depicted in Figure 3 ). The height (H) may vary between 90 to 100 mm and the width (W) may vary between 205 to 220 mm. As shown in Figures 1, 2 and 5 , the body 100 includes a face portion 200, contoured recesses 300, padding part 350, filler part 400, outer portion 500 and seam 600.

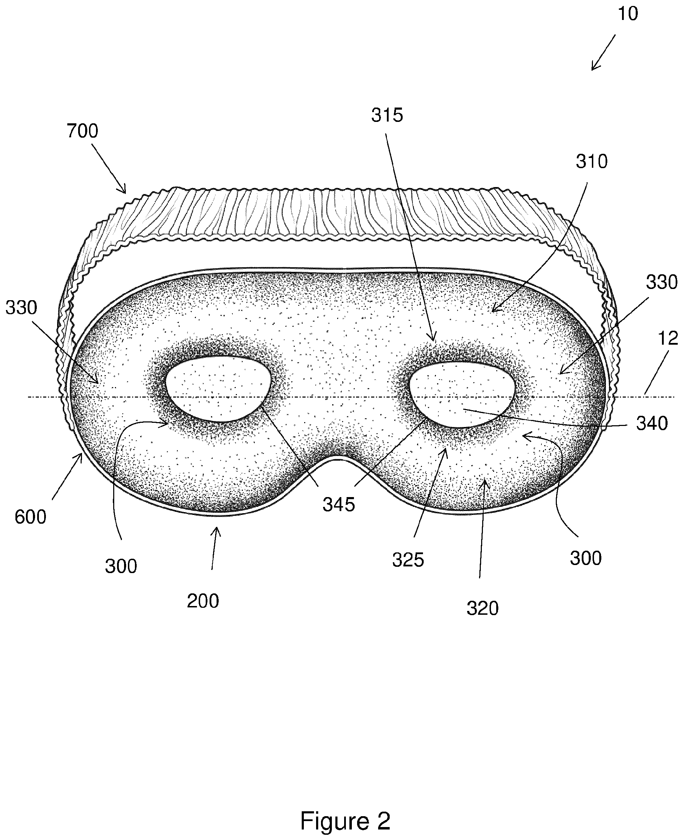

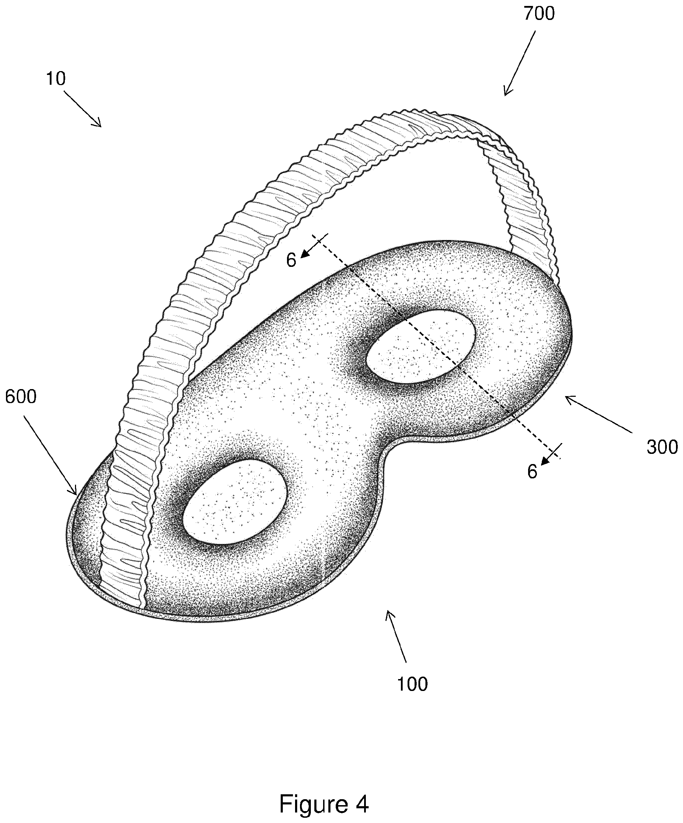

The face portion 200 is shown further in Figures 2 to 6 . The face portion 200 is intended to be placed against the face of a wearer when worn in a normal fashion. The face portion 200 is, preferably, made from silk and, in further embodiments, it may be made from satin or other materials. The silk absorbs less face cream compared to (for example) cotton. The silk also creates less friction on a wearer's skin compared to cotton. Satin can also have a low friction finish. The silk is preferably a (6A) long fibre mulberry silk. The thickness of the silk is preferably approximately 22 momme. The face portion 200 is formed from a separate layer and, as detailed below, is stitched together with other components of the body 100 to form an external surface.

The face portion 200 conforms to the recesses 300 (or vice versa). The contoured recesses 300 are associated with the eyes of a wearer when the sleep mask 10 is worn. That is, the contoured recesses 300 align with a wearer's eyes during use. As detailed further below, the shape of the contoured recesses 300 ensure that there is no or limited contact of the eyelashes or eyelash extensions to the mask 10 itself and in turn to the wearer's pillow or bedding. The contoured recesses 300 include an upper ridge 310, an upper taper 315, a lower ridge 320, a lower taper 325, a side ridge 330 and a valley portion 340. The contoured recesses 300 are moulded into shape. In other words, the contoured recesses 300 are formed into specific, gradual shapes that suitable fit onto a wearer's face.

The upper ridge 310 is configured engage against a forehead of the wearer. The upper ridge 310 is substantially located above the retaining part 700. The upper ridge 310 extends adjacent to an upper portion of the valley portion 340. The lower ridge 320 extends adjacent to a lower portion of the valley portion 340. The lower ridge 320 is configured to engage against a cheek area of the wearer. The lower ridge 320 extends further away from the outer portion 500 of the body 100, in a direction towards the eyes of the wearer, compared to the upper ridge 310. This assists with the body 100 conforming to a variety of faces and, for example, blocking out sunlight whilst eyelash extensions are not compromised. The approximate width (F) of the ridges is approximately 28mm to 29mm and this may vary ±7.5mm.

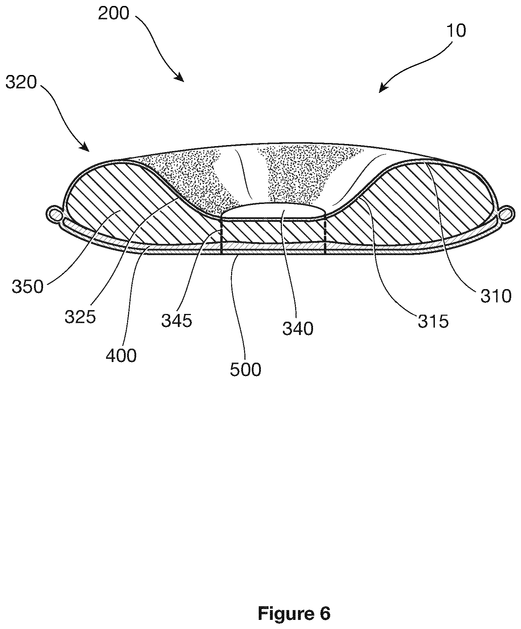

An upper taper portion 315 is formed between the upper ridge 310 and the valley portion 340. A lower taper portion 325 is also formed between the lower ridge 320 and the valley portion 340. The distance (T) between the upper end of the taper portion 315 and the upper end of taper portion 325 is approximately 49 mm. This distance (T) may vary between 38 and 58 mm. The lower taper 325 is formed with a shallower angle than the upper taper 315. This again assists with suitably conforming the body 100 to multiple wearers. In addition, as detailed further below and evident in Figure 6 , the face portion 200 is at least partly offset from the padding part 350 in the lower taper portion 325. This offset provides suitable leeway for body 100 conforming to a wearer. The taper portions 315, 325 provide a gradual transition between the ridges 310, 320 and the valley portion 340. This transition is between 30 to 60 degrees.

A side ridge 330 is located between the upper ridge 310 and lower ridge 320. As will be appreciated by a person skilled in the art, the ridges 310, 320 and 330 form one uniform ridge but the structure of each part has been detailed. The side ridges 330 are located adjacent the retaining part 700. The side ridges 330 assists in blocking out light to the right and left of a wearer.

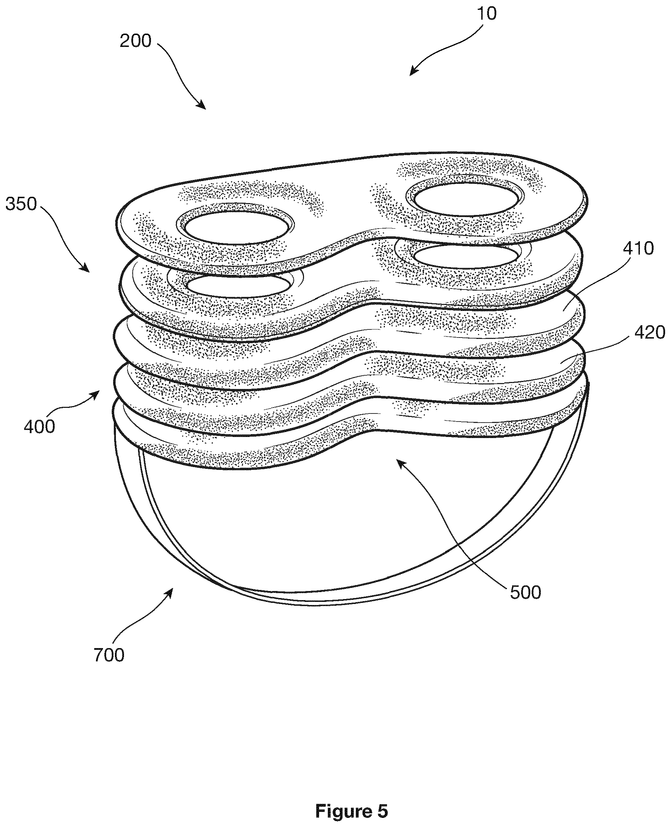

As evident in Figure 5 and 6 , the valley portion 340 is substantially flat. The valley portion 340 of each recess 300 is substantially oval in shape. The shape of the valley portion 340 is formed by a seam within the contoured recess 300. That is, the shape of the valley portion 340 is formed by stitching through the face portion 200 to the padding part 350. The valley stitching is shown as 345. The valley portion 340 is biased to one side of a longitudinal centre line 12 extending across the body 100. As shown in the embodiment of Figure 2 , a greater proportion of the valley portion 340 is found above the longitudinal centre line 12 towards the upper ridge 310. The valley portion 340 of this embodiment is asymmetrical. In this manner, the shape of the valley portion 340 is more suitable for matching the shape of an eye and eyelashes (including extensions if present) of a wearer. The valley portion 340 has a height (P) of approximately 26 mm and a width (A) of approximately 39 mm. This height (P) may vary between 21 to 31mm and the width (A) may vary between 34 mm to 44 mm. The depth of the recesses 300 is approximately 15 mm but this can vary between 10 mm to 25 mm.

Figure 5 illustrates an exploded view the sleep mask 10, and shows each layer of the mask 10. Depicted adjacent the face portion 200 is a padding part 350. The padding part 350 may be made from foam. The padding part 350 assists in forming the shape of the contoured recesses 300. That is, the recesses 300 are substantially created by connecting the face portion 200 and the padding part 350. In this regard, the padding part 350 (and subsequently the contoured recesses 300) is of the depth and width required to accommodate various lengths and shapes of eyelashes and accommodate a variety of face sizes and shapes. It will be appreciated that the padding part 350 may be provided in one or multiple pieces.

The padding part 350 is polyester in this embodiment. However, in further embodiments, the padding part 350 may be a memory foam. The memory foam may include polyurethane. The padding part 350 has an average density of approximately 32.6 kg/m<3> and this may vary between approximately 28kg/m<3> to 38kg/m<3>. To this end, as shown in Figure 6 , the density of the padding part 350 associated with the lower ridge 320 and the upper ridge 310 is lower compared to the density of the padding part 350 associated with the valley portion 340. In other words, a middle portion of the padding part 350 is more dense compared to its outer part(s). The surface hardness of the padding part 350 is approximately 42-45 using an asker durometer type F. During manufacturing, the padding part 350 is compressed in a mould, with different elevated temperatures on its front and back surfaces, to obtain these desired mechanical properties. In this regard, the material of the padding part 350 (its relative composition and density) has been carefully selected and manufactured to ensure: that the compression of the foam to the user's face is not uncomfortable or has a rigid feeling; and the foam has sufficient rigidity such the mask may block light from the user's external environment.

As shown in Figure 5 , a filler part 400 is found outboard from the padding part 350 in a direction away from the face portion 200. In further embodiments, a filler part 400 may be located between the padding part 350 and the face portion 200. The filler part 400 may take the form of a layer. The filler part 400 is designed to provide comfort. The filler part 400 assists in bridging contours between the different shape of the padding part 350 compared to a preferred continuous shape of the outer portion 500. The filler part 400 includes a liner 410 and a filler 420. The filler 420 is made from tussah silk. The liner 410 is made from mulberry silk. In further embodiments, the filler 420 and the liner 410 may be made from satin.

The outer portion 500 (further shown in Figure 5 ) forms an outer layer. The outer portion 500 is found the furthest from the face when worn by a user. The outer portion 500 is provided as a smooth layer of material with no stitching present. In a similar manner to the face portion 200, the outer portion 500 is made from silk. A seam 600 connects the face portion 200 and the outer portion 500 together. This seam 600 can take the form of a stitching portion. The seam 600 runs around the border of the body 100. The retaining part 700 is also preferably stitched to the seam 600. In this regard, during production, the seam 600 is used to: i) contain the padding part 350 and filler part 400 between the face portion 200 and the outer portion 500; and ii) connect the retaining part 700 to the body 100. The stitching 345 also assists in forming / shaping the contoured recesses 300.

As indicated above, the sleep mask 10 is held in place over the wearer's eyes with a retaining part 700. The retaining part 700 extends from a first side of the body 100 to a second opposing side of the body 100. The retaining part 700 is configured to extend around the back of the head of the wearer. The retaining part 700 is substantially located to one side of the axis 12. The retaining part 700 is made of a flexible material, preferably elastic. The flexible material is covered by silk (and in further embodiments may be covered by satin). The retaining part 700 has a crumpled and/or pleated look.

During use, a wearer stretches the retaining part 700 to place it around their head. The body 100 is positioned on the wearer's face such that the nose portion 150 sits on the wearer's nose. In this position, at least the ridges 310, 320, 330 distance the valley portion 340 in a position away from the wearer's face such that, if eye lash extensions are used, they do not engage with the recess portions 300.

With the above in mind, the sleep mask 10 or certain embodiments may be capable of achieving certain advantages. These advantages may include some or all of the following: reducing contact of all or portions of a wearer's eyelash extensions and/or long eyelashes with the sleep mask 10, the wearer's pillowcase and/or other bedding, thereby protecting the shape and bond of any eyelash extensions or long eyelashes; reducing contact between the wearer's eyes and the mask 10, thereby increasing user comfort for those users who do not like to sleep with a traditional sleep mask which compresses/touches their eyes; simultaneously providing function of a traditional sleep mask, including by blocking light to the wearer's external environment; and improving wearer comfort via composition of each component of the product.

In this specification, the terms 'comprises', 'comprising', 'includes', 'including', or similar terms are intended to mean a non-exclusive inclusion, such that a method, system or apparatus that comprises a list of elements does not include those elements solely, but may well include other elements not listed.

The above description relating to embodiments of the present disclosure is provided for purposes of description to one of ordinary skill in the related art. It is not intended to be exhaustive or to limit the disclosure to a single disclosed embodiment. As mentioned above, numerous alternatives and variations to the present disclosure will be apparent to those skilled in the art from the above teaching. Accordingly, while some alternative embodiments have been discussed specifically, other embodiments will be apparent or relatively easily developed by those of ordinary skill in the art.

### Item List:

| **Item No** | **Item** |
|---|---|
| Sleep Mask | 10 |
| Longitudinal Centre Line | 12 |
| Body | 100 |
| Top Edge of the Body | 110 |
| Sides of the Body | 120 |
| Curve/Bottom Portion of the Body | 130 |
| Nose Portion | 150 |
| Face Portion | 200 |
| Recess | 300 |
| Upper Ridge | 310 |
| Upper Taper | 315 |
| Lower Ridge | 320 |
| Lower Taper | 325 |
| Side Ridge | 330 |
| Valley Portion | 340 |
| Recess Stitching | 345 |
| Padding Part | 350 |
| Filler Part | 400 |
| Liner | 410 |
| Filler | 420 |
| Outer Portion | 500 |
| Seam | 600 |
| Retaining Part | 700 |
| | |

| Dimensions | |
|---|---|
| Approximate Width of Valley Portion | A |
| Approximate Width of Body | W |
| Approximate Width of Upper Ridge | F |
| Approximate Height of Body | H |
| Approximate Height of Valley Portion | P |
| Approximate Height between upper ends of Tapered Portions | T |

## Claims

1. A mask (10) including:
a body (100) adapted to cover eyes of a wearer, the body having:
an outer portion (500);
a face portion (200);
one or more padding parts (350); and
two contoured recesses (300) to be associated with the eyes of a wearer when the mask (10) is worn, the contoured recesses (300) include a valley portion (340), wherein the valley portion (340) is substantially flat and/or asymmetrical and/or biased to one side of a longitudinal centre line (12) extending across the body (100); and
a retaining part (700) connected to the body (100), the retaining part (700) being configured to retain the body (100) to the wearer,
wherein the one or more padding parts (350) are located between the outer portion (500) and the face portion (200), and the contoured recesses (300) are present in at least one of the or each padding part (350),
**characterised in that**:
a recess seam (345) extends through the face portion (200) into the one or more padding portions (350) to define the valley portion (340) and wherein the recess seam (345) includes stitching.

2. The mask (10) of claim 1, wherein the two contoured recesses (300) are configured to avoid contact with eyelash extensions, and wherein, preferably, the two contoured recesses (300) are configured to avoid contact with eyes and/or long eyelashes.

3. The mask (10) of claim 1 or 2, wherein the one or more padding parts (350) are configured to form the shape of the contoured recesses (300).

4. The mask (10) of any one of claims 1 to 3, wherein the outer portion (500) and/or the face portion (200) form a layer wherein, preferably, the layer is at least partly made of silk or satin.

5. The mask (10) of any one of claims 1 to 4, wherein the contoured recesses (300) include an upper ridge (310) and a lower ridge (320), and preferably wherein the lower ridge (320) extends further away from an outer portion (500) of the body (100), in a direction towards the eyes of the wearer, compared to the upper ridge (310).

6. The mask (10) of claim 5, wherein the density of the one or more padding parts (350) associated with at least part of the lower ridge (320) or the upper ridge (310) is lower compared to the density of the one or more padding parts (350) associated with the valley portion (340) and, wherein, preferably, the one or more padding parts (350) include a density of approximately 28kg/m³ to 38kg/m³.

7. The mask (10) of any one of claims 5 to 6, wherein the face portion (200) is at least partly offset from the one or more padding parts (350) in a taper portion (325) between the lower ridge (320) of the contoured recesses (300) and the valley portion (340).

8. The mask (10) of any one of claims 1 to 7, wherein a seam portion (600) connects the face portion (200) and the outer portion (500) together with the one or more padding parts (350) therebetween, and wherein, preferably:
the seam portion (600) connects the outer portion (500) to the face portion (200) to retain a filler part (400) and the one or more padding parts (350); and/or
the seam portion (600) includes stitching.

9. The mask (10) of any one of claims 1 to 8, wherein the body (100) includes a filler part (400), wherein:
The filler part (400) takes the form of a layer; and/or
the filler part (400) includes a filler (420) and/or a liner (410),
wherein, preferably the filler (420) and/or the liner (410) include silk or satin, preferably wherein the silk includes tussah silk or mulberry silk.

10. The mask (10) of claim 9, wherein the filler part (400) is:
configured to provide comfort to the wearer and/or improve the appearance of the mask (10); and/or
located between the one or more padding parts (350) and at least one of the face portion (200) or the outer portion (500);
wherein, preferably, the filler part (400) is configured to:
bridge contours between the one or more padding parts (350) and at least one of the outer portion (500) or the face portion (200); and/or
provide support to the outer portion (500) to allow the outer portion (500) to form a shape that is different to the one or more padding parts (350).

11. The mask (10) of any one of claims 1 to 10, wherein the one or more padding parts (350) are made from foam, preferably, wherein:
the one or more padding parts (350) include polyester and/or polyurethane; and/or
the surface hardness of the one or more padding parts (350) measures 32 to 55 on an asker durometer type F.

12. A method of producing a mask (10), the method including the steps of:
moulding two contoured recesses (300) in one or more padding parts (350) to form part of a body (100), the contoured recesses (300) to be associated with eyes of a wearer when the mask (10) is worn, the contoured recesses (300) including a valley portion (340), wherein the valley portion (340) is substantially flat and/or asymmetrical and/or biased to one side of a longitudinal centre line (12) extending across the body (100);
locating the one or more padding parts (350) between an outer portion (500) and a face portion (200);
connecting the outer portion (500) to the face portion (200); and
connecting a retaining part (700) to the body (100),
**characterised in that**:
a recess seam (345) is formed by stitching through the face portion (200) into the one or more padding portions (350) to define the valley portion (340).

13. The method of claim 12, wherein:
the step of connecting the retaining part (700) to the body (100) includes stitching the retaining part (700) to the body (100), wherein, preferably, the step of connecting the face portion (200) to the outer portion (500) includes stitching the two together; and/or
wherein the method further includes locating a filler part (400) between the one or more padding parts (350) and at least one of the face portion (200) or the outer portion (500), wherein, preferably, the step of locating the filler part (400) includes locating it between the one or more padding parts (350) and the outer portion (500) of the body (100).

## Patentansprüche

1. Maske (10) mit:
einem Körper (100), der dazu ausgebildet ist, die Augen eines Trägers zu bedecken, wobei der Körper aufweist:
einen äußeren Teil (500);
einen Gesichtsteil (200);
ein oder mehrere Polsterteile (350); und
zwei konturierte Ausnehmungen (300), welche den Augen eines Trägers zuzuordnen sind, wenn die Maske getragen wird, wobei die konturierten Ausnehmungen (300) einen Muldenbereich (340) aufweisen, wobei der Muldenbereich (340) im Wesentlichen flach und/oder symmetrisch und/oder zu einer Seite einer sich über den Körper (100) erstreckenden Längsmittellinie (12) versetzt ist; und
ein Halteteil (700), das mit dem Körper (100) verbunden ist, wobei das Halteteil (700) dazu ausgebildet ist, den Körper (100) an dem Träger zu halten,
wobei sich das eine oder die mehreren Polsterteile (350) zwischen dem äußeren Teil (500) und dem Gesichtsteil (200) befinden, und die konturierten Ausnehmungen (300) in mindestens einem oder in jedem Polsterteil (350) vorhanden sind,
**dadurch gekennzeichnet,**
**dass** sich ein Ausnehmungssaum (345) durch den Gesichtsteil (200) in das eine oder die mehreren Polsterteile (350) erstreckt, um den Muldenbereich zu bilden, und wobei der Ausnehmungssaum (345) eine Naht aufweist.

2. Maske (10) nach Anspruch 1, bei welcher die beiden konturierten Ausnehmungen (300) dazu ausgebildet sind, Kontakt mit Wimpernverlängerungen zu vermeiden, und wobei vorzugsweise die beiden konturierten Ausnehmungen (300) dazu ausgebildet sind, Kontakt mit Augen und/oder langen Wimpern zu vermeiden.

3. Maske (10) nach Anspruch 1 oder 2, bei welcher das eine oder die mehreren Polsterteile (350) dazu ausgebildet sind, die Form der konturierten Ausnehmungen (300) zu bilden.

4. Maske (10) nach einem der Ansprüche 1 bis 3, bei welcher der äußere Teil (500) und/oder der Gesichtsteil (200) eine Schicht bilden, wobei vorzugsweise die Schicht zumindest teilweise aus Seide oder Satin besteht.

5. Maske (10) nach einem der Ansprüche 1 bis 4, bei welcher die konturierten Ausnehmungen (300) einen oberen Wulst (310) und einen unteren Wulst (320) aufweisen, und wobei sich vorzugsweise der untere Wulst (320) im Vergleich mit dem oberen Wulst (310) weiter von einem äußeren Teil (500) des Körpers (100) weg in Richtung der Augen des Trägers erstreckt.

6. Maske (10) nach Anspruch 5, bei welcher die Dichte des einen oder der mehreren Polsterteile (350), die zumindest einem Teil des unteren Wulsts (320) oder des oberen Wulsts (310) zugeordnet sind, im Vergleich mit der Dichte des einen oder der mehreren Polsterteile (350), die dem Muldenbereich (340) zugeordnet sind, geringer ist, und wobei vorzugsweise das eine oder die mehreren Polsterteile (350) eine Dichte von ungefähr 28 kg/m³ bis 38 kg/m³ aufweisen.

7. Maske (10) nach einem der Ansprüche 5 bis 6, bei welcher der Gesichtsteil (200) in einem Verjüngungsbereich (325) zwischen der unteren Wulst (320) der konturierten Ausnehmungen (300) und dem Muldenbereich (340) zumindest teilweise gegenüber dem einen oder den mehreren Polsterteilen (350) versetzt ist.

8. Maske (10) nach einem der Ansprüche 1 bis 7, bei welcher ein Saumbereich (600) den Gesichtsteil (200) und den äußeren Teil (500) miteinander verbindet, wobei das eine oder die mehreren Polsterteile (350) dazwischen angeordnet sind, und wobei vorzugsweise:
der Saumbereich (600) den äußeren Teil (500) mit dem Gesichtsteil (200) verbindet, um ein Füllteil (400) und das eine oder die mehreren Polsterteile (350) zurückzuhalten; und/oder
der Saumbereich (600) eine Naht aufweist.

9. Maske (10) nach einem der Ansprüche 1 bis 8, bei welcher der Körper (100) ein Füllteil (400) aufweist, wobei:
das Füllteil (400) die Form einer Schicht aufweist; und/oder
das Füllteil (400) ein Füllmaterial (420) und/oder eine Auskleidung (410) aufweist,
wobei vorzugsweise das Füllmaterial (420) und/oder die Auskleidung (410) Seide oder Satin aufweist, wobei die Seide vorzugsweise Tussahseide oder Maulbeerseide aufweist.

10. Maske (10) nach Anspruch 9, bei welcher das Füllteil (400),
dazu ausgebildet ist, dem Träger Komfort zu vermitteln und/oder das Erscheinungsbild der Maske (10) zu verbessern; und/oder
zwischen dem einen oder den mehreren Polsterteilen (350) und dem Gesichtsteil (200) und/oder dem äußeren Teil (500) angeordnet ist;
wobei das Füllteil (400) vorzugsweise dazu ausgebildet ist,
Konturen zwischen dem einen oder den mehreren Polsterteilen (350) und dem äußeren Teil (500) und/oder dem Gesichtsteil (200) zu überbrücken; und/oder
den äußeren Teil (500) zu stützen, um dem äußeren Teil (500) zu ermöglichen, eine Form zu bilden, die von dem einen oder den mehreren Polsterteilen (350) verschieden ist.

11. Maske (10) nach einem der Ansprüche 1 bis 10, bei welcher das eine oder die mehreren Polsterteile (350) aus Schaummaterial bestehen, wobei vorzugsweise:
das eine oder die mehreren Polsterteile (350) Polyester und/oder Polyurethan aufweisen; und/oder
die Oberflächenhärte des einen oder der mehreren Polsterteile (350) 32 bis 44 auf einem Asker Durometer Typ F beträgt.

12. Verfahren zur Herstellung einer Maske (10), wobei das Verfahren die folgenden Schritte aufweist:
Formen zweier konturierter Ausnehmungen (300) in einem oder mehreren Polsterteilen (350) zur Bildung eines Teils eines Körpers (100), welche den Augen eines Trägers zuzuordnen sind, wenn die Maske getragen wird, wobei die konturierten Ausnehmungen (300) einen Muldenbereich (340) aufweisen, wobei der Muldenbereich (340) im Wesentlichen flach und/oder symmetrisch und/oder zu einer Seite einer sich übe den Körper (100) erstreckenden Längsmittellinie (12) versetzt ist;
Anordnen des einen oder der mehreren Polsterteile (350) zwischen einem äußeren Teil (500) und einem Gesichtsteil (200);
Verbinden des äußeren Teils (500) mit dem Gesichtsteil (200); und
Verbinden eines Rückhalteteils (700) mit dem Körper (100),
**dadurch gekennzeichnet,**
**dass** ein Ausnehmungssaum (345) durch Nähen durch den Gesichtsteil (200) in das eine oder die mehreren Polsterteile (350) gebildet wird, um den Muldenbereich (340) zu bilden.

13. Verfahren nach Anspruch 12, bei welchem
der Schritt des Verbindens des Rückhalteteils (700) mit dem Körper (100) das Nähen des Rückhalteteils (700) an den Körper (100) aufweist, wobei vorzugsweise der Schritt des Verbindens des Gesichtsteils (200) mit dem äußeren Teil (500) das Vernähen der beiden miteinander aufweist; und/oder
wobei das Verfahren ferner das Anordnen eines Füllteils (400) zwischen dem einen oder den mehreren Polsterteilen 350 und dem Gesichtsteil (200) und/oder dem äußeren Teil (500) aufweist, wobei vorzugsweise der Schritt des Anordnens des Füllteils (400) das Anordnen desselben zwischen dem einen oder den mehreren Polsterteilen (350) und dem äußeren Teil (500) des Körpers (100) aufweist.

## Revendications

1. Masque (10) comprenant :
un corps (100) adapté pour couvrir les yeux d'un porteur, le corps ayant :
une partie extérieure (500) ;
une partie visage (200);
une ou plusieurs parties de rembourrage (350); et
deux renfoncements galbés (300) à associer aux yeux d'un porteur lorsque le masque (10) est porté, les renfoncements galbés (300) comprenant une partie creuse (340), la partie creuse (340) étant essentiellement plate et/ou asymétrique et/ou biaisée vers un côté d'une ligne centrale longitudinale (12) s'étendant à traves le corps (100) ; et
une partie de retenue (700) reliée au corps (100), la partie de retenue (700) étant configurée pour retenir le corps (100) au porteur,
lesdites une ou plusieurs parties de rembourrage (350) étant situées entre la partie extérieure (500) et la partie visage (200) et les renfoncements galbés (300) étant présents dans au moins une ou dans chacune des parties de rembourrage (350),
**caractérisé en ce**
**qu'**une couture de renfoncement (345) s'étend à travers la partie visage (200) dans lesdites une ou plusieurs parties de rembourrage (350) pour définir la partie creuse (340) et la couture de renfoncement (345) comprenant du piquage.

2. Masque (10) selon la revendication 1, les deux renfoncements galbés (300) étant configurés pour éviter un contact avec des extensions de cils et, de préférence, les deux renfoncements galbés (300) étant configurés pour éviter un contact avec les yeux et/ou des cils longs.

3. Masque (10) selon la revendication 1 ou 2, lesdites une ou plusieurs parties de rembourrage (350) étant configurées pour créer la forme des renfoncements galbés (300).

4. Masque (10) selon l'une des revendications 1 à 3, la partie extérieure (500) et/ou la partie visage (200) formant une couche, de préférence, la couche étant faite au moins en partie en soie ou satin.

5. Masque (10) selon l'une des revendications 1 à 4, les renfoncements galbés (300) comprenant une arête supérieure (310) et une arête inférieure (320) et, de préférence, l'arête inférieure (320) s'étendant, comparé à l'arête supérieure (310), plus loin d'une partie extérieure (500) du corps (100) dans une direction vers les yeux du porteur.

6. Masque (10) selon la revendication 5, la densité d'une ou de plusieurs parties de rembourrage (350) associée(s) à au moins une partie de l'arête inférieure (320) ou de l'arête supérieure (310) étant inférieure, comparée à la densité d'une ou de plusieurs parties de rembourrage (350)associée(s) à la partie creuse (340), et, de préférence, lesdites une ou plusieurs parties de rembourrage (350) ayant une densité d'environ 28 kg/m³ à 38 kg/m³.

7. Masque (10) selon l'une des revendications 5 à 6, la partie visage (200) étant au moins partiellement décalée desdites une ou plusieurs parties de rembourrage (350) dans une partie conique (325) entre l'arête inférieure (320) des renfoncements galbés (300) et la partie creuse (340).

8. Masque (10) selon l'une des revendications 1 à 7, une partie de suture (600) reliant la partie visage (200) à la partie extérieure (500) avec une ou plusieurs parties de rembourrage (350) entre elles, et de préférence :
la partie de suture (600) reliant la partie extérieure (500) à la partie visage (200) pour retenir une partie de remplissage (400) et lesdites une ou plusieurs parties de rembourrage (350), et/ou
la partie de suture (600) comprenant du piquage.

9. Masque (10) selon l'une des revendications 1 à 8, le corps (100) comprenant une partie de remplissage (400),
la partie de remplissage (400) ayant la forme d'une couche ; et/ou
la partie de remplissage (400) comprenant une charge (420) et/ou une doublure (410),
la charge (420) et/ou la doublure (410) comprenant de la soie ou du satin, de préférence, la soie comprend de la soie tussah ou de la soie de mûre.

10. Masque (10) selon la revendication 9, la partie de remplissage (400) étant :
configurée pour donner du confort au porteur et/ou pour améliorer l'aspect du masque (10) ; et/ou
située entre lesdites une ou plusieurs parties de rembourrage (350) et au moins une des parties de visage (200) ou la partie extérieure (500) ;
de préférence, la partie de remplissage (400) étant configurée pour :
combler des contours entre lesdites une ou plusieurs parties de rembourrage (350) et au moins une parmi la partie extérieure (500) ou la partie visage (200) ; et/ou
donner un support à la partie extérieure (500) pour permettre à la partie extérieure (500) de créer une forme qui est différente desdites une ou plusieurs parties de rembourrage (350).

11. Masque (10) selon l'une des revendications 1 à 10, lesdites une ou plusieurs parties de rembourrage (350) étant faites en mousse, de préférence :
lesdites une ou plusieurs parties de rembourrage (350) comprenant du polyester et/ou du polyuréthane ; et/ou
la dureté de surface desdites une ou plusieurs parties de rembourrage (350) étant de 32 à 55 sur un duromètre Asker de type F.

12. Procédé de fabriquer un masque (10), le procédé comprenant les étapes de :
mouler deux renfoncements galbés (300) dans une ou plusieurs parties de rembourrage (350) pour former une partie d'un corps (100), les renfoncements galbés (300), à associer aux yeux d'un porteur lorsque le masque (10) est porté, les renfoncements galbés (300) comprenant une partie creuse (340), la partie creuse (340) étant essentiellement plate et/ou asymétrique et/ou biaisée vers un côté d'une ligne centrale longitudinale (12) s'étendant à travers le corps (100) ;
disposer lesdites une ou plusieurs parties de rembourrage (350) entre une partie extérieure (500) et une partie visage (200) ;
connecter la partie extérieure (500) à la partie visage (200) ; et
connecter une partie de retenue (700) au corps (100),
**caractérisé en ce**
**qu'**une couture de renfoncement (345) est formée en piquant à travers la partie visage (200) dans lesdites une ou plusieurs parties de rembourrage (350) pour définir la partie creuse (340).

13. Procédé selon la revendication 12,
l'étape de connecter la partie de retenue (700) au corps (100) comprenant coudre la partie de retenue (700) au corps (100), de préférence, l'étape de connecter la partie visage (200) à la partie extérieure (500) comprenant coudre les deux ensembles ; et/ou
le procédé comprenant en outre disposer une partie de remplissage (400) entre lesdites une ou plusieurs parties de rembourrage (350) et au moins une parmi la partie visage (200) ou la partie extérieure (500), de préférence, l'étape de disposer la partie de remplissage (400) comprenant le disposer entre lesdites une ou plusieurs parties de rembourrage (350) et la partie extérieure (500) du corps (100).
